# FASCICULE DE BREVET EUROPEEN

(11) **EP 0 999 796 B1**
(45) Date de publication et mention de la délivrance du brevet: **16.04.2003**
(21) Numéro de dépôt: 98940342.3
(22) Date de dépôt: 27.07.1998
(51) Int. Cl.: A61B 17/70, A61B 17/80

(54) **IMPLANT, NOTAMMENT PLAQUE ANTERIEURE CERVICALE**
IMPLANTAT, INSBESONDERE KNOCHENPLATTE FÜR DIE VORDERE SEITE DER WIRBELSÄULE
IMPLANT, IN PARTICULAR FRONT CERVICAL PLATE

(30) Priorité: 28.07.1997 FR 9709579
(43) Date de publication de la demande: 17.05.2000
(73) Titulaire: Dimso Distribution Medicale du Sud Ouest, 33610 Cestas (FR)
(72) Inventeur: BACCELLI, Christian, F-33650 Saint Médard d'Eyrans (FR)
(74) Mandataire: Le Forestier, Eric
(86) Numéro de dépôt international: FR9801653
(87) Numéro de publication internationale: WO99004718

(56) Documents cités:
- EP-A- 0 599 766
- EP-A- 0 705 572
- DE-A- 4 132 021
- FR-A- 2 728 454
- US-A- 2 443 363
- US-A- 5 607 428
- US-A- 5 616 142

## Description

La présente invention a trait d'une façon générale aux systèmes de fixation pour l'ostéosynthèse du rachis, et concerne en particulier une nouvelle plaque dite plaque cervicale, destinée à solidariser de manière particulière au moins deux vertèbres cervicales.

Les techniques de fusion osseuse au niveau cervical sont bien connues et couramment pratiquées dans des indications tels que le rachis dégénératif, les hernies discales ou plus généralement divers cas de traumatologie ou de tumeurs.

Ces techniques reposent sur le principe d'une discectomie (à savoir l'ablation d'un disque), simple ou multiple, éventuellement associée à une corporectomie (ablation d'un corps vertébral), également simple ou multiple;

Les espaces laissés libres par de telles ablations sont généralement comblés par des greffons osseux taillés aux dimensions souhaitées.

A ce sujet, on sait que pour pouvoir obtenir une fusion osseuse à partir d'un greffon, il est nécessaire que celui-ci soit soumis à un état de contraintes en compression. Cette compression est généralement obcenue par un léger surdimensionnement du greffon, associé à une distraction per-opératoire entre les structures osseuses cervicales adjacents.

Le document FR-A-2 728 454 décrit un implant conforme au préambule de la revendication 1.

On connaît déjà par le document US-A-5 616 142 un implant possédant une plaque pleine en deux parties coulissantes, dont chacune possède des trous pour des vis d'ancrage dans deux vertèbres adjacentes.

Cette plaque pleine permet de contenir les matériaux introduits entre les deux vertèbres, tels que des blocs d'hydroxyapatite, des greffons osseux ou encore des substituts osseux.

Par ailleurs, la faculté qu'ont les deux parties de coulisser l'une par rapport à l'autre permet de s'adapter aisément à différents espacements entre vertèbres.

Cette plaque connue est toutefois désavantageuse en ce qu'elle présente un risque de jeu entre les deux parties, qui peut faciliter le desserrement accidentel des vis.

En outre, cette possibilité de coulissement rend l'implant incapable de maintenir un écartement souhaité entre les deux vertèbres dans la direction longitudinale du rachis, si bien que la compression initiale du greffon osseux peut se trouver affectée, avec en conséquence une mauvaise qualité ou rapidité de fusion, voire une fusion totalement défectueuse.

La présente invention vise à pallier ces inconvénients de l'état de la technique et à proposer un implant, utilisable notamment comme plaque cervicale antérieure, dont la pose soit sensiblement simplifiée et dans lequel on puisse tirer parti d'une plaque en deux éléments coulissants pour établir et maintenir une compression de greffons osseux introduits entre deux vertèbres sur lesquelles l'implant vient s'ancrer.

Ainsi la présente invention propose un implant pour ostéosynthèse du rachis, comprenant une plaque destinée à être fixée à l'aide de vis de fixation osseuse à deux vertèbres telles que des vertèbres cervicales, notamment par voie antérieure, caractérisé en ce que la plaque comporte deux éléments comportant chacun au moins un trou pour une vis de fixation osseuse, chacun des éléments étant susceptible de coulisser par rapport à l'autre dans une direction correspondant à un rapprochement ou à un éloignement des vertèbres l'une par rapport à l'autre, et lesdits éléments étant pourvus de moyens pour limiter la course de coulissement mutuel entre ceux-ci et pour bloquer lesdits éléments dans une position mutuelle donnée.

Des aspects préférés, mais non limitatifs, de l'implant selon l'invention sont les suivants :
- les deux éléments sont disposés tête-bêche.
- les deux éléments sont identiques.
- chaque élément comporte une première partie terminale comportant le ou les trous pour vis de fixation osseuse, et une seconde partie terminale possédant une ouverture oblongue dont le grand axe définit la direction de coulissement, ladite ouverture étant traversée par un élément saillant solidaire d'une partie intermédiaire de l'autre élément.
- chaque élément saillant consiste en une vis constituant l'un desdits moyens de blocage.
- ladite partie intermédiaire de chaque élément se situe dans le prolongement et dans la continuité de ladite. première partie terminale.
- ladite seconde partie terminale de chaque élément se trouve décalée en hauteur par rapport à ladite première partie terminale et à ladite partie intermédiaire, de manière à recouvrir la partie intermédiaire de l'autre élément.
- chaque élément comprend en outre une partie de liaison plus étroite entre sa partie intermédiaire et sa seconde partie terminale.
- lesdites parties de liaison des deux éléments délimitent chacune une partie d'un passage traversant de la plaque.
- ledit passage traversant est oblong et possède à sa base un épaulement périphérique.
- ledit épaulement est en biseau.
- l'implant comprend en outre au moins une vis d'ancrage auxiliaire engagée dans ledit passage traversant.
- la partie de liaison de chaque élément définit au moins un épaulement apte à coopérer avec la seconde partie terminale ou avec la partie intermédiaire de l'autre élément, de manière à définir une butée de coulissement des deux éléments.
- chaque vis de fixation osseuse comporte un filet intermédiaire destiné à coopérer avec un filet correspondant prévu dans le trou pour vis associé.
- chaque vis de fixation osseuse comporte une tête de contour généralement conique et pourvue d'une pluralité de branches séparées par des interstices d'orientation générale axiale, la conicité de la tête étant apte à bloquer la vis contre la rotation par friction de ladite tête dans le trou associé.
- ladite tête possède une rainure apte à coopérer avec une partie intérieurement saillante formée dans le trou associé, pour réaliser un blocage axial.
- au moins certains des trous pour vis de fixation osseuse présentent un axe non perpendiculaire à un plan général de la plaque.

L'invention propose également un jeu d'implants, caractérisé en ce qu'il comprend une pluralité d'implants tels que définis ci-dessus, avec des éléments de plaque présentant différents nombres de trous pour vis de fixation osseuse et différentes orientations de trous pour vis, et une pluralité de vis de fixation osseuse, et en ce que les différents éléments de plaque sont visuellement différenciés.

D'autres aspects, buts et avantages de la présente invention apparaîtront mieux à la lecture de la description détaillée suivante de formes de réalisation préférées de celle-ci, donnée à titre d'exemple et faite en référence aux dessins annexés, sur lesquels :
la figure 1 est une vue en perspective d'ensemble d'un implant cervical selon l'invention,
la figure 2 est une vue en perspective de l'implant en l'absence des vis de fixation osseuse,
la figure 3 est une vue en perspective éclatée de l'ensemble de l'implant de la figure 1,
la figure 4 est une vue en perspective, à échelle agrandie, d'une partie d'une vis de fixation osseuse utilisable avec l'implant des figures 1 à 3,
la figure 5 est une vue en perspective d'une partie de l'implant et d'une vis de fixation osseuse selon une autre forme de réalisation, avant vissage de la vis dans une vertèbre,
la figure 6 est une vue identique à celle de la figure 5, après vissage dans une vertèbre,
la figure 7 est une vue en perspective d'un implant selon les figures 1 à 3 complété par une vis intermédiaire, et
la figure 8 est une vue en perspective, selon une autre orientation, de l'implant de la figure 7.

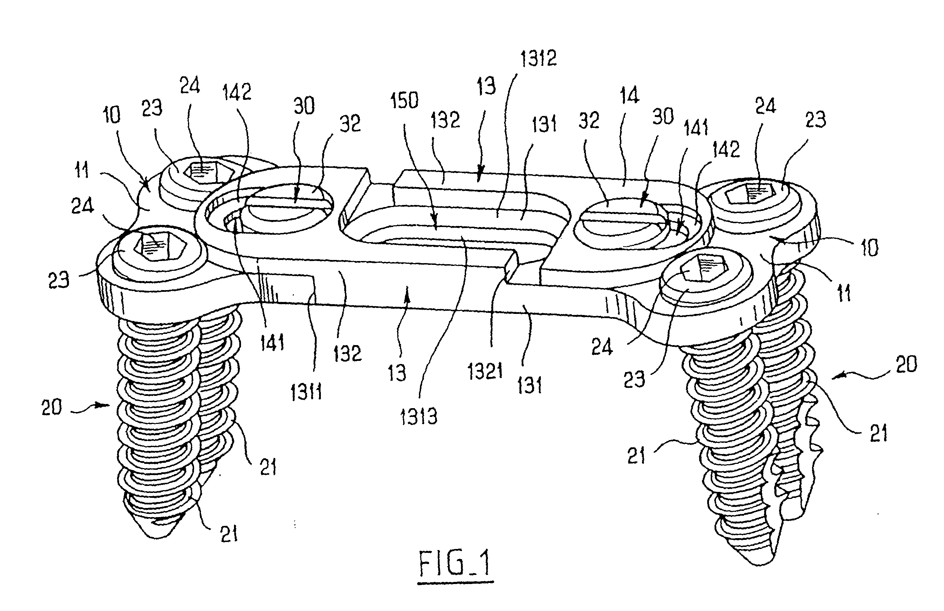

En référence au dessin, et tout d'abord aux figures 1 à 3, on a représenté un implant formant plaque antérieure cervicale pour la chirurgie rachidienne, qui comprend deux éléments de plaque identiques 10, 10 aptes à coopérer avec des vis d'ancrage osseux.

Chaque élément de plaque comprend une partie terminale 11 pour ancrage osseux, une partie intermédiaire 12 de guidage, une partie intermédiaire 13 de liaison et une partie terminale 14 de guidage.

La partie 11, en forme de plaque mince de contours arrondis, possède côte-à-côte deux ouvertures traversantes 111 généralement circulaires, à l'intérieur desquelles est ménagé un filet 1111 de verrouillage, ces ouvertures 111 étant destinées à recevoir chacune une vis 20 d'ancrage osseux 20.

La partie intermédiaire 12 s'étendant dans le prolongement de la partie 11, sur une largeur moins étendue, et possède un alésage traversant taraudé 121.

La partie de liaison 13 est généralement rectiligne et sensiblement plus étroite que les parties 11 et 12, en s'étendant dans le prolongement d'un bord latéral de ladite partie 12. Cette partie 13 possède une région 131 situé dans le prolongement de la partie 12 en terme de hauteur, et une région 132 surélevée par rapport à la région 131. Un premier décrochement transversal 1311 marque l'extrémité de la région 131 à l'opposé de la partie 12, tandis qu'un second décrochement transversal 1321 marque l'extrémité de la région 132 du côté de la partie 12.

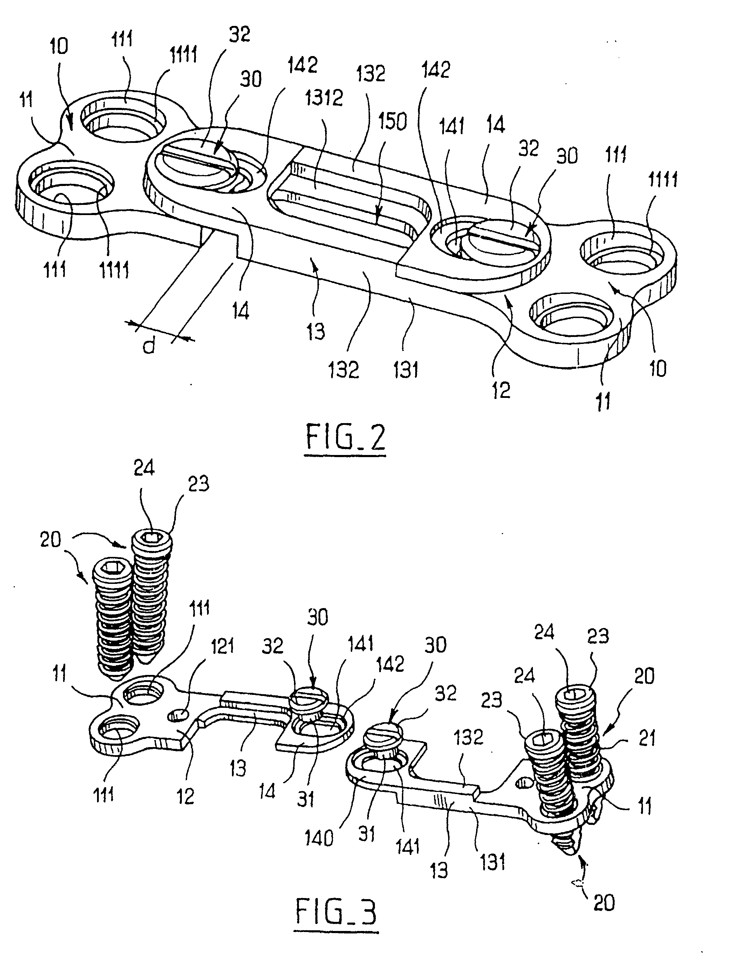

On observera ici que la région inférieure 131 de la partie de liaison 13 possède, du côté intérieur, une largeur plus importante que la région 132, en étant délimitée par une face intérieure de base 1313 et par une face intermédiaire en biseau 1312 réunissant la face 1313 à la face intérieure de la région 132. Comme le montre en particulier la figure 3, ce profil particulier adopté par l'intérieur de la région 131 se prolonge selon deux quarts de cercle, du côté de la partie 12 et du côté de la partie 14.

La partie 14 se situe, en terme de hauteur, dans le prolongement de la région supérieure 132 de la partie 13, et présente une largeur identique à celle de la partie 12. Elle se termine par une face d'extrémité en demi-cercle, et est traversée par une ouverture oblongue 141 dont le grand axe est sur l'axe longitudinal de l'élément de plaque 10. Cette ouverture 141 est entièrement entourée par un gradin en creux 142.

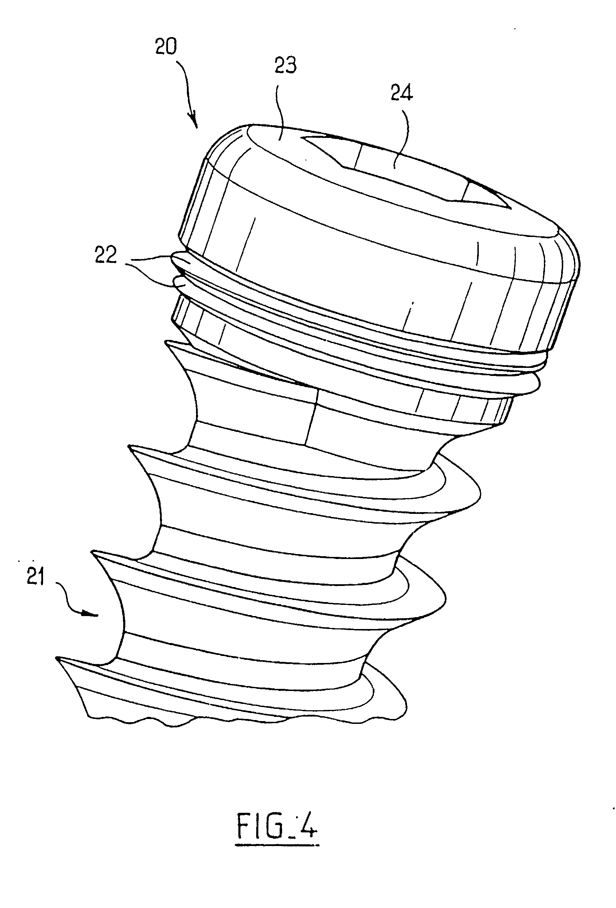

Comme le montre en particulier la figure 4, chaque vis 20 d'ancrage osseux comprend principalement une tige filetée 21 destinée à pénétrer dans une vertèbre cervicale, et une tête plus large 23 dans laquelle est pratiquée une empreinte en creux 24 à six pans pour un outil de vissage. Il est par ailleurs prévu à la transition entre la partie filetée 21 et la tête 23, un filetage 22 de pas sensiblement intérieur à celui du filetage 21, s'étendant sur une hauteur très limitée et destiné à coopérer avec le filet 1111 prévu dans l'ouverture 111 associée de l'élément de plaque, à des fins expliquées plus loin.

Enfin, la vis 20 possède dans la région de sa pointe un évidement concave 25 à bords vifs, destiné à faciliter l'attaque de la matière osseuse par les filets.

Enfin l'implant selon l'invention possède deux autres vis 30, possédant chacune une courte partie filetée 31 lui permettant d'être vissée dans un alésage associé 121 de l'élément de plaque 10, en traversant librement l'ouverture oblongue 141 de l'autre élément de plaque 10, et une tête 32 possédant une rainure pour le vissage, et dont la largeur est très légèrement inférieure à la largeur entre les gradins opposés prévus autour de ladite ouverture 141.

Le montage des deux éléments de plaque 10, 10 s'effectue en disposant ces deux éléments tête-bêche, et en plaçant la partie terminale 14 de chacune au-dessus de la partie intermédiaire 12 de l'autre.

Les deux vis 30 sont alors mises en place à travers les ouvertures respectives 141 et vissées dan les alésages respectifs 121, sans toutefois les bloquer.

On comprend qu'à partir de cet instant, les deux éléments de plaque 10, 10 sont solidarisés l'un à l'autre en conservant un seul degré de liberté en coulissement entre deux butées selon la direction axiale. La course de ce coulissement, indiquée en d sur la figure 2, est définie en particulier par la longueur des ouvertures oblongues 141, et est choisie égale à quelques millimètres, par exemple deux millimètres. On observera ici que les butées de fin de course de ce coulissement mutuel sont définies non seulement par les extrémités des ouvertures oblongues 141 contre lesquelles viennent s'appuyer les vis 30, mais éventuellement, dans le sens inverse, par la venue en appui des épaulements 1311, 1321 de chaque élément 10, décrits plus haut, respectivement contre les faces en vis-à-vis de la partie intermédiaire 12 et de la partie terminale 14 de l'autre élément 10.

On observe également que, lors de l'assemblage des deux éléments de plaque 10, 10, on définit dans la région centrale de la plaque ainsi obtenue un passage traversant oblong en direction axiale, désigné par la référence 150, qui est défini conjointement par les parties intermédiaires de liaison 13, 13 des deux éléments 10, 10. Ce passage possède dans sa région de base un épaulement oblique périphérique continu défini conjointement par les faces 1312 prévues sur les deux éléments de plaque, qui rétrécit la section dudit passage, à des fins expliquées plus loin.

La pose par le chirurgien s'effectue en introduisant tout d'abord, dans l'espace intervertébral situé entre les deux vertèbres cervicales dans lesquelles l'implant doit être ancré, des greffons osseux destinés, à terme, à la fusion osseuse entre les deux vertèbres.

Ensuite, l'implant étant préalablement préparé en introduisant les quatre vis d'ancrage osseux 20 dans leurs quatre ouvertures respectives 111, le chirurgien les visse par paires dans deux vertèbres adjacentes.

Pendant cette opération, les deux éléments de plaque 10, 10 peuvent être soit bloqués mutuellement à l'aide des vis 30 dans une position distractée au maximum (position de la figure 2), soit libres de coulisser l'un par rapport à l'autre.

A la fin du serrage des vis d'ancrage osseux 20, les filets intermédiaires 22 de celles-ci sont aptes à coopérer avec les filets 1111 prévus dans leurs ouvertures respectives 111 pour assurer leur blocage à fond de filet, et éviter ainsi leur desserrement.

Ensuite, les vis 30 étant si nécessaires desserrées, le chirurgien effectue à l'aide d'un outillage approprié un rapprochement des vertèbres, qui a pour effet de comprimer les greffons osseux et de faciliter la prise de ces greffons. Au cours de ce mouvement, les deux éléments de plaque 10, 10 coulissent l'un par rapport à l'autre. Lorsque le degré de compression requis est. atteint, l'ensemble est maintenu dans cette position et les deux vis 30 sont serrées pour bloquer la plaque cervicale dans cette position pour assurer durablement la compression des greffons.

On notera ici que, grâce au double recouvrement des deux éléments de plaque 10, d'une part entre la partie intermédiaire 12 de l'une et la partie terminale 14 de l'autre et, en sens inverse et à distance, entre la partie intermédiaire 12 de l'autre et la partie terminale 14 de l'une, on obtient finalement une plaque présentant une excellente rigidité, notamment vis-à-vis des flexions.

On observera également ici, comme le montrent en particulier les figures 1 et 3, que les vis d'ancrage osseux 20 peuvent être orientées de façon inclinée par rapport à la normale au plan générale de la plaque cervicale 10, 10.

Plus précisément, on peut prévoir que les ouvertures traversantes 111 des éléments 10 aient leurs axes inclinés, soit vers l'extérieur de la plaque, soit vers l'intérieur, et aussi bien dans sa direction longitudinale que dans sa direction transversale, pour adapter l'orientation des tiges filetées 21 des vis 20 à la configuration osseuse du patient.

Cette inclinaison peut être typiquement comprise, dans une direction ou dans l'autre, entre 0° et 15°.

Dans la pratique, on propose au chirurgien différents jeux de plaques avec différents nombres et/ou différentes inclinaisons des ouvertures 111.

Il est à noter ici que les vis 20 à filet intermédiaire 22, en coopération avec des trous pour vis 111 pourvus d'un filet associé 1111, peuvent être utilisées non seulement avec des plaques en deux parties selon la présente invention, mais plus généralement dans toute sorte d'implant destiné à être traversé par une vis d'ancrage osseux.

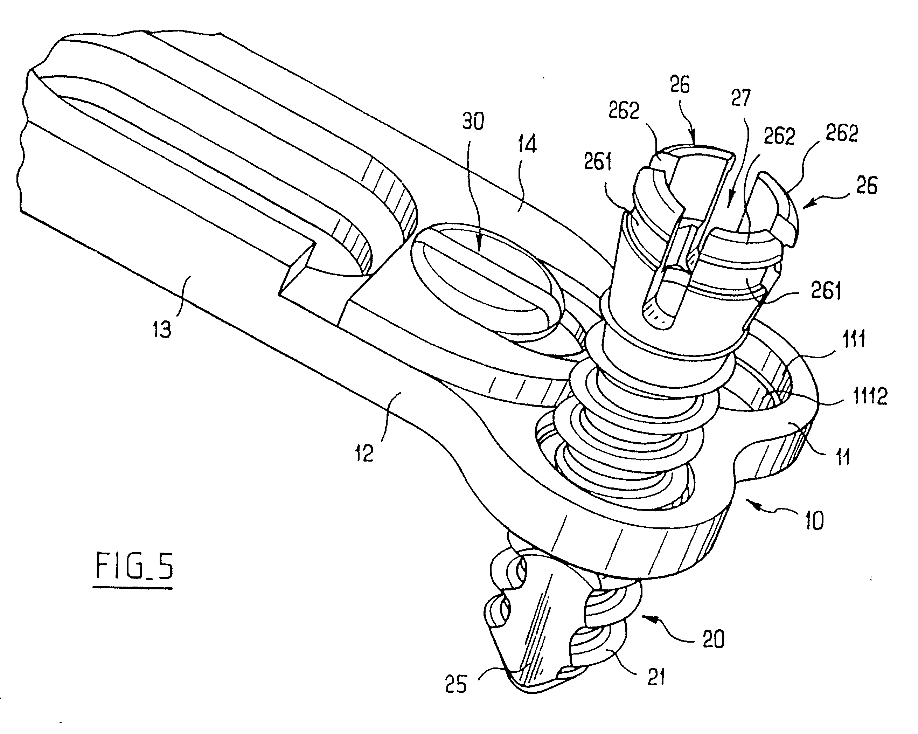

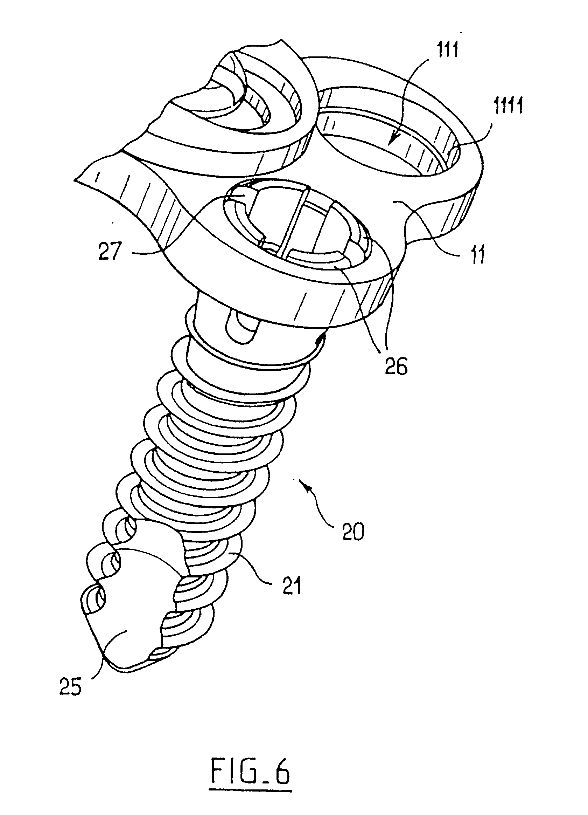

Les figures 5 et 6 illustrent une autre forme de réalisation des vis d'ancrage, désignées en 20'.

Ces vis comprennent, outre le filetage osseux 21 et l'évidement 25, une tête creuse 23' de section circulaire et légèrement tronconique, dont le diamètre extérieur est voisin de celui de la partie filetée. La conicité de la tête 23' est telle que sa section diminue de son extrémité externe vers la tige filetée 21,

Cette tête cylindrique possède quatre branches 26 régulièrement espacées en direction circonférentielle, qui sont séparées par des interstices 27 orientés axialement.

Chaque branche possède sur sa face externe une rainure circonférentielle 261, et une dent d'accrochage 262 est ainsi définie entre ladite rainure et l'extrémité libre de la tête.

La tête possède par ailleurs, de façon non visible, une empreinte pour outils de vissage prévue dans la région de racine des quatre branches 26.

Par ailleurs, les ouvertures 111 prévues dans les éléments de plaque 10 pour recevoir les vis 20 possèdent non plus un filet, mais un gradin, indiqué en 1112 sur la figure 5, prévu à la base de l'ouverture respective 111, dont la dimension axiale est sensiblement égale à celle de la rainure 261 de chacune des branches, et qui fait saillie vers l'intérieur.

La vis 20' est mise en place par vissage dans l'os et, à mesure de sa progression, la tête 23' va s'engager dans l'ouverture 111, les branches 26 se déformant alors élastiquement vers l'intérieur, sous l'effet de la sollicitation exercée par le gradin 1112, jusqu'à ce que ce dernier vienne se loger intimement dans chacune des quatre rainures 261.

Par ailleurs, la dimension et l'angle de conicité de la tête 23' et la dimension de chaque ouverture associée 111 sont telles qu'à la fin du serrage, l'effort de frottement entre la surface extérieure de la tête et la paroi de l'ouverture associée 111 soit suffisant pour éviter le desserrement de la vis de fixation osseuse 20'.

La forme de réalisation illustrée sur les figures 5 et 6 est avantageuse en ce qu'en fin de montage, la face externe de chaque vis 20' est sensiblement affleurante avec la face extérieure de la partie 11 de la plaque 20, ce qui diminue l'encombrement global de l'ensemble.

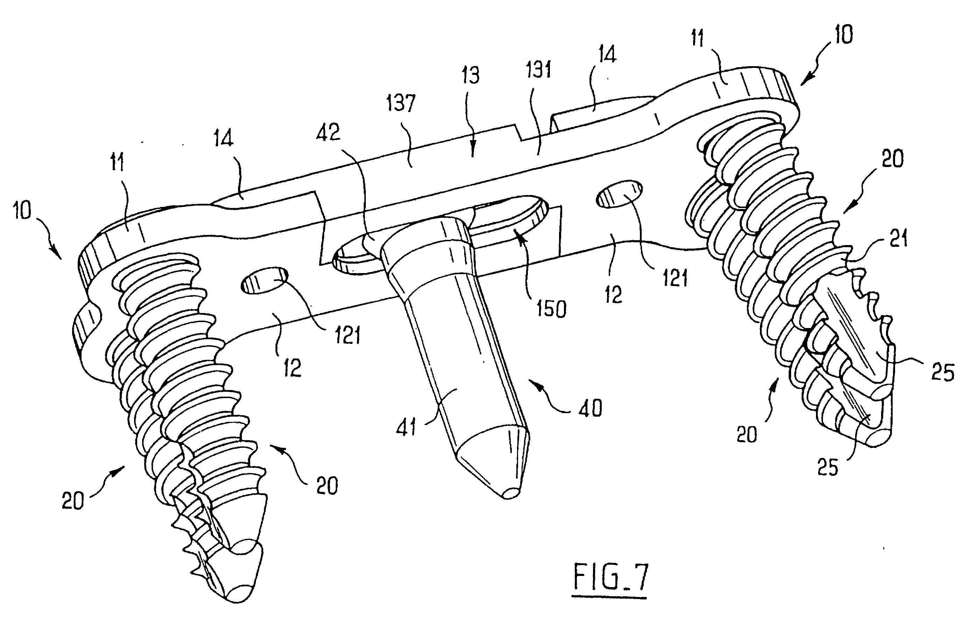

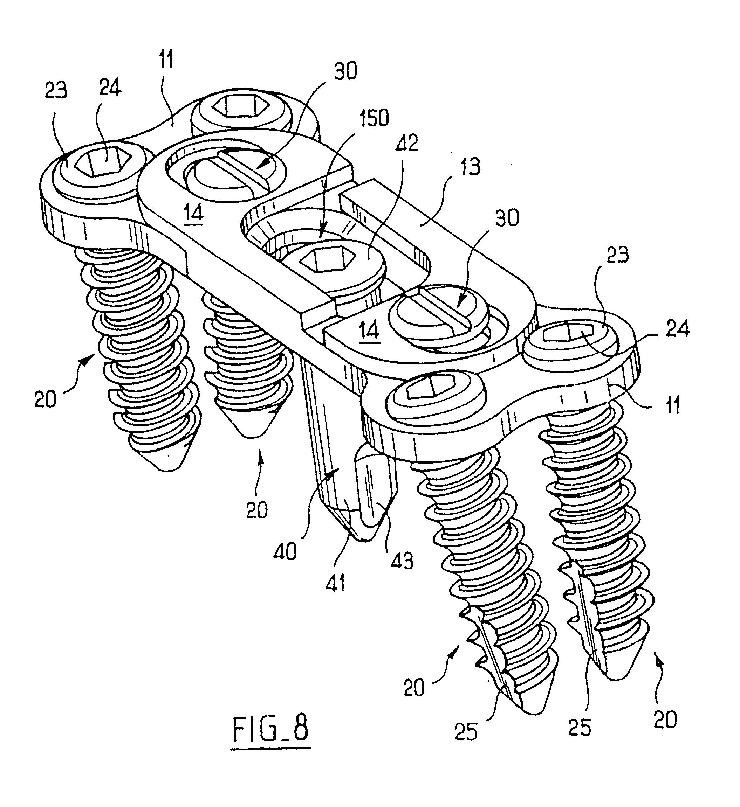

En référence maintenant aux figures 7 et 8, on a illustré le cas où l'implant formant plaque cervicale décrit ci-dessus est complété par une vis, schématiquement représentée en 40, qui est destinée à pénétrer dans le greffon osseux placé entre les deux vertèbres, en étant mise en place à travers le passage 150 défini par les deux éléments de plaque 10 lors de leur assemblage.

Une telle vis comprend une tige 41, dont le filetage n'a pas été représenté par souci de simplification, et une tête plus large 42, pourvue d'une empreinte en creux à six pans pour outil de vissage.

Le diamètre hors tout de la tige filetée 41 est inférieur à la largeur minimale du passage (au niveau des régions de base 131 des parties de liaison 13 des éléments 10), tandis que le diamètre de sa tête est plus grand que sa largeur maximale (au niveau des régions 132 desdites parties 13).

De la sorte, la tête 42 de la vis 40 peut ne pas déborder vers l'extérieur par rapport au contour externe de la plaque assemblée 10, 10, comme le montre la figure 5.

Cette figure montre également que la partie filetée 41 possède au voisinage de sa pointe un évidement 43 facilitant l'attaque des filets.

L'adjonction d'une telle vis 40 permet d'améliorer la cohésion mécanique de l'ensemble, la croissance osseuse s'effectuant en prise avec les filets de cette vis.

En outre, selon la longueur du passage oblong 150, on peut prévoir de disposer dans celui-ci deux vis 40 ou même davantage.

On observera également que la tête 41 de la vis 40 possède une partie de base 411 en forme de portion de sphère, ce qui permet à la vis 40 d'être aisément inclinée par rapport à la normale au plan général de la plaque, de manière à s'adapter à la configuration de l'espace intervertébral recevant les greffons osseux. L'angulation possible peut par exemple atteindre 20°.

Naturellement, les différents composants des implants décrits ci-dessus sont réalisés en un matériau biocompatible tel qu'un alliage de titane ou un acier inoxydable.

Bien entendu, la présente invention n'est nullement limitée aux formes de réalisation décrites et représentées, mais l'homme du métier saura y apporter toute variante ou modification conforme à son esprit.

En particulier, on peut prévoir un nombre de vis d'ancrage osseux 20 différent de deux par élément 10.

Par ailleurs, on propose de préférence au chirurgien une boîte comportant un jeu d'une pluralité d'implants et de vis d'ancrage, avec des éléments de plaque 10 présentant par exemple 1, 2 3 ou 4 trous 111 pour vis de fixation, et des éléments de plaque présentant différentes orientations de ces trous 111 par rapport au plan général de la partie 11 des éléments.

Dans la boîte, ces différents éléments 10 sont par exemple différenciés par des codes de couleurs ou analogues.

## Revendications

1. Implant pour ostéosynthèse du rachis, comprenant une plaque (10, 10) apte à être fixée à l'aide de vis de fixation osseuse (20) à deux vertèbres telles que des vertèbres cervicales, comportant deux éléments (10, 10) comportant chacun au moins un trou (111) apte à recevoir une vis de fixation osseuse (20), chacun des éléments étant susceptible d'effectue un coulissement par rapport à l'autre dans une direction correspondant à un rapprochement ou à un éloignement desdits éléments l'un par rapport à l'autre, **caractérisé en ce que** lesdits éléments sont pourvus de moyens (141, 30) aptes à bloquer lesdits éléments dans une position mutuelle quelconque dans une course dudit coulissement.

2. Implant selon la revendication 1, **caractérisé en ce que** les deux éléments (10, 10) sont disposés tête-bêche.

3. Implant selon l'une des revendications 1 et 2, **caractérisé en ce que** les deux éléments (10, 10) sont identiques.

4. Implant selon l'une des revendications 2 et 3, **caractérisé en ce que** chaque élément comporte une première partie terminale (11) comportant le ou les trous (111) pour vis de fixation osseuse (20), et une seconde partie terminale (14) possédant une ouverture oblongue (141) dont le grand axe définit la direction de coulissement, ladite ouverture étant traversée par un élément saillant (30) solidaire d'une partie intermédiaire (12) de l'autre élément.

5. Implant selon la revendication 4, **caractérisé en ce que** chaque élément saillant consiste en une vis (30) constituant l'un desdits moyens de blocage.

6. Implant selon l'une des revendications 4 et 5, **caractérisé en ce que** ladite partie intermédiaire (12) de chaque élément se situe dans le prolongement et dans la continuité de ladite première partie terminale (11).

7. Implant selon la revendication 6, **caractérisé en ce que** ladite seconde partie terminale (14) de chaque élément se trouve décalée en hauteur par rapport à ladite première partie terminale (11) et à ladite partie intermédiaire (12), de manière à recouvrir la partie intermédiaire de l'autre élément.

8. Implant selon l'une des revendications 5 à 7, **caractérisé en ce que** chaque élément comprend en outre une partie de liaison plus étroite (13) entre sa partie intermédiaire (12) et sa seconde partie terminale (14).

9. Implant selon la revendication 8, **caractérisé en ce que** lesdites parties de liaison (13, 13) des deux éléments délimitent chacune une partie d'un passage traversant (150) de la plaque.

10. Implant selon la revendication 9, **caractérisé en ce que** ledit passage traversant (150) est oblong et possède à sa base un épaulement périphérique (1312).

11. Implant selon la revendication 10, **caractérisé en ce que** ledit épaulement (1312) est en biseau.

12. Implant selon l'une des revendications 1 à 11, **caractérisé en ce qu'**il comprend en outre au moins une vis d'ancrage auxiliaire (40).

13. Implant selon l'une des revendications 8 à 12, **caractérisé en ce que** la partie de liaison (13) de chaque élément définit au moins un épaulement (1321, 1311) apte à coopérer avec la seconde partie terminale (14) ou avec la partie intermédiaire (12) de l'autre élément, de manière à définir une butée de coulissement des deux éléments.

14. Implant selon l'une des revendications 1 à 13, **caractérisé en ce que** chaque vis de fixation osseuse (20) comporte un filet intermédiaire (22) destiné à coopérer avec un filet correspondant (1111) prévu dans le trou pour vis associé.

15. Implant selon l'une des revendications 1 à 13, **caractérisé en ce que** chaque vis de fixation osseuse (20') comporte une tête (23') de contour généralement tronconique et pourvue d'une pluralité de branches (26) séparées par des interstices (27) d'orientation générale axiale, la conicité de la tête étant apte à bloquer la vis contre la rotation par friction de ladite tête dans le trou associé (111).

16. Implant selon la revendication 15, **caractérisé en ce que** ladite tête (23') possède une rainure (261) apte à coopérer avec une partie intérieurement saillante (1112) formée dans le trou associé (111), pour réaliser un blocage axial.

17. Implant selon l'une des revendications 1 à 16, **caractérisé en ce que** au moins certains des trous (111) pour vis de fixation osseuse présentent un axe non perpendiculaire à un plan général de la plaque.

18. Jeu d'implants, **caractérisé en ce qu'**il comprend une pluralité d'implants selon l'une des revendications 1 à 17, avec des éléments de plaque présentant différents nombres de trous pour vis de fixation osseuse et différentes orientations de trous pour vis, et une pluralité de vis de fixation osseuse, et **en ce que** les différents éléments de plaque sont visuellement différenciés.

## Claims

1. Implant for osteosynthesis of the spine, comprising a plate (10, 10) capable of being fixed to two vertebrae, such as cervical vertebrae, with the aid of bone fixation screws (20), comprising two elements (10, 10), each including at least one hole (111) capable of receiving a bone fixation screw (20), each of the elements being able to perform sliding relative to the other in a direction corresponding to a closing together or distancing of the said elements relative to one another, **characterized in that** the said elements are provided with means (141, 30) capable of blocking the said elements in any mutual position in the course of the said sliding.

2. Implant according to Claim 1, **characterized in that** the two elements (10, 10) are arranged head to foot.

3. Implant according to one of Claims 1 and 2, **characterized in that** the two elements (10, 10) are identical.

4. Implant according to one of Claims 2 and 3, **characterized in that** each element includes a first end part (11) including the hole or holes (111) for bone fixation screws (20), and a second end part (14) having an oblong opening (141) whose main axis defines the direction of sliding, the said opening being traversed by a projecting element (30) integral with an intermediate part (12) of the other element.

5. Implant according to Claim 4, **characterized in that** each projecting element consists of a screw (30) constituting one of the said blocking means.

6. Implant according to one of Claims 4 and 5, **characterized in that** the said intermediate part (12) of each element is situated in the extension and continuation of the said first end part (11).

7. Implant according to Claim 6, **characterized in that** the said second end part (14) of each element is situated offset in height relative to the said first end part (11) and to the said intermediate part (12) in such a way as to cover the, intermediate part of the other element.

8. Implant according to one of Claims 5 to 7, **characterized in that** each element additionally comprises a narrower joining part (13) between its intermediate part (12) and its second end part (14).

9. Implant according to Claim 8, **characterized in that** the said joining parts (13, 13) of the two elements each delimit part of a through-passage (150) of the plate.

10. Implant according to Claim 9, **characterized in that** the said through-passage (150) is oblong and has at its base a peripheral shoulder (1312).

11. Implant according to Claim 10, **characterized in that** the said shoulder (1312) is bevelled.

12. Implant according to one of Claims 1 to 11, **characterized in that** it additionally comprises at least one auxiliary anchoring screw (40).

13. Implant according to one of Claims 8 to 12, **characterized in that** the joining part (13) of each element defines at least one shoulder (1321, 1311) able to cooperate with the second end part (14) or with the intermediate part (12) of the other element, in such a way as to define a limit stop for the sliding of the two elements.

14. Implant according to one of Claims 1 to 13, **characterized in that** each bone fixation screw (20) includes an intermediate thread (22) intended to cooperate with a corresponding thread (1111) provided in the hole for the associated screw.

15. Implant according to one of Claims 1 to 13, **characterized in that** each bone fixation screw (20') includes a head (23') of generally frustoconical contour and provided with a plurality of branches (26) which are separated by interstices (27) of generally axial orientation, the conicity of the head being able to block the screw against rotation by means of the friction of the said head in the associated hole (111).

16. Implant according to Claim 15, **characterized in that** the said head (23') has a groove (261) able to cooperate with an inwardly projecting part (1112) formed in the associated hole (111) for producing axial blocking.

17. Implant according to one of Claims 1 to 16, **characterized in that** at least some of the holes (111) for bone fixation screws have an axis not perpendicular to a general plane of the plate.

18. Set of implants, **characterized in that** it comprises a plurality of implants according to one of Claims 1 to 17, with plate elements which have different numbers of holes for bone fixation screws and different orientations of holes for screws, and a plurality of bone fixation screws, and **in that** the different plate elements are visibly differentiated.

## Patentansprüche

1. Implantat für die Osteosynthese der Wirbelsäule, mit einer Platte (10, 10), die dazu geeignet ist, mit Hilfe einer Befestigungsschraube für Knochen (20) an zwei Wirbeln, wie Halswirbeln, befestigt zu werden, und die zwei Elemente (10, 10) mit jeweils mindestens einem Loch (111) aufweist, das dazu geeignet ist, eine Befestigungsschraube für Knochen (20) aufzunehmen, wobei jedes der Elemente bezüglich des anderen Elementes eine Gleitbewegung in einer Richtung ausführen kann, welche einer Annäherung oder einer beabstandeten Anordnung der Elemente zueinander entspricht, **dadurch gekennzeichnet, dass** die Elemente mit Vorrichtungen (141, 30) versehen sind, die dazu geeignet sind, die Elemente in irgendeiner Position zueinander im Verlauf der Gleitbewegung zu blockieren.

2. Implantat gemäß Anspruch 1, **dadurch gekennzeichnet, dass** die beiden Elemente (10, 10) kopfüber zueinander angeordnet sind.

3. Implantat gemäß einem der Ansprüche 1 und 2, **dadurch gekennzeichnet, dass** die beiden Elemente (10, 10) identisch ausgebildet sind.

4. Implantat gemäß einem der Ansprüche 2 und 3, **dadurch gekennzeichnet, dass** jedes Element einen ersten Endbereich (11) mit dem oder den Löchern (111) für eine Befestigungsschraube für Knochen (20) und einen zweiten Endbereich (14) umfasst, der eine Längsöffnung (141) aufweist, deren Hauptachse die Gleitrichtung definiert, wobei diese Öffnung von einem vorspringenden Element (30) durchquert wird, das einstückig mit einem Zwischenbereich (12) des anderen Elementes ausgebildet ist.

5. Implantat gemäß Anspruch 4, **dadurch gekennzeichnet, dass** jedes vorspringende Element aus einer Schraube (30) besteht, welche eine der Blockiervorrichtungen bildet.

6. Implantat gemäß einem der Ansprüche 4 und 5, **dadurch gekennzeichnet, dass** der Zwischenbereich (12) jedes Elementes in der Verlängerung und der Fortsetzung des ersten Endbereiches (11) angeordnet ist.

7. Implantat gemäß Anspruch 6, **dadurch gekennzeichnet, dass** der zweite Endbereich (14) jedes Elementes bezüglich des ersten Endbereiches (11) und des Zwischenbereiches (12) in der Höhe versetzt derart angeordnet ist, dass er den Zwischenbereich des anderen Elementes überdeckt.

8. Implantat gemäß einem der Ansprüche 5 bis 7, **dadurch gekennzeichnet, dass** jedes Element außerdem einen schmaleren Verbindungsbereich (13) zwischen - seinem Zwischenbereich (12) und seinem zweiten Endbereich (14) umfasst.

9. Implantat gemäß Anspruch 8, **dadurch gekennzeichnet, dass** die Verbindungsbereiche (13, 13) der beiden Elemente jeweils einen Durchgangsbereich (150), der die Platte durchquert, begrenzen.

10. Implantat gemäß Anspruch 9, **dadurch gekennzeichnet, dass** der Durchgangsbereich (150) länglich ausgebildet ist und an seiner Basis einen Umfangsansatz (1312) aufweist.

11. Implantat gemäß Anspruch 10, **dadurch gekennzeichnet, dass** der Ansatz (1312) abgeschrägt ausgebildet ist.

12. Implantat gemäß einem der Ansprüche 1 bis 11, **dadurch gekennzeichnet, dass** es außerdem wenigstens eine Hilfsverankerungsschraube (40) aufweist.

13. Implantat gemäß einem der Ansprüche 8 bis 12, **dadurch gekennzeichnet, dass** der Verbindungsbereich (13) jedes Elementes wenigstens einen Ansatz (1321, 1311) definiert, der dazu geeignet ist, mit dem zweiten Endbereich (14) oder mit dem Zwischenbereich (12) des anderen Elementes derart zusammenzuwirken, dass er einen Anschlag für die Gleitbewegung der beiden Elemente definiert.

14. Implantat gemäß einem der Ansprüche 1 bis 13, **dadurch gekennzeichnet, dass** jede Knochenbefestigungsschraube (20) ein Zwischengewinde (22) aufweist, welches dazu bestimmt ist, mit einem entsprechenden Gewinde (1111) zusammenzuwirken, das in dem Loch für die zugehörige Schraube vorgesehen ist.

15. Implantat gemäß einem der Ansprüche 1 bis 13, **dadurch gekennzeichnet, dass** jede Knochenbefestigungsschraube (20') einen Kopf (23') mit einer allgemein kegelstumpfartigen Kontur aufweist, der mit einer Vielzahl von Abzweigungen (26) versehen ist, die durch Zwischenräume (27) mit allgemein axialer Ausrichtung voneinander getrennt sind, wobei die konische Ausbildung des Kopfes dazu geeignet ist, die Schraube gegen die Drehung durch Reibung des Kopfes in dem zugehörigen Loch (111) zu blockieren.

16. Implantat gemäß Anspruch 15, **dadurch gekennzeichnet, dass** der Kopf (23') eine Rille (261) aufweist, die dazu in der Lage ist, mit einem nach innen vorspringenden Bereich (1112) zusammenzuwirken, der in dem zugehörigen Loch (111) ausgebildet ist, um eine axiale Blockierung zu erreichen.

17. Implantat gemäß einem der Ansprüche 1 bis 16, **dadurch gekennzeichnet, dass** wenigstens bestimmte Löcher (111) für die Knochenbefestigungsschraube eine Achse aufweisen, die nicht rechtwinklig zu einer allgemeinen Ebene der Platte liegt.

18. Implantatsatz, **dadurch gekennzeichnet, dass** er eine Vielzahl von Implantaten gemäß einem der Ansprüche 1 bis 17 mit Plattenelementen aufweist, welche unterschiedliche Anzahlen von Löchern für Knochenbefestigungsschrauben und unterschiedliche Ausrichtungen der Schraubenlöcher aufweisen, und er eine Vielzahl von Knochenbefestigungsschrauben umfasst, und dass die verschiedenen Plattenelemente visuell unterschiedlich gestaltet sind.
